# EUROPEAN PATENT SPECIFICATION

(11) **EP 4 436 640 B1**
(45) Date of publication and mention of the grant of the patent: **31.12.2025**
(21) Application number: 22826820.7
(22) Date of filing: 25.11.2022
(51) Int. Cl.: A61M 11/00, A61M 15/00

(54) **MEDICAMENT DELIVERY DEVICE**
MEDIKAMENTENABGABEVORRICHTUNG
DISPOSITIF D'ADMINISTRATION DE MÉDICAMENTS

(30) Priority: 25.11.2021 GB 202117043
(43) Date of publication of application: 02.10.2024
(73) Proprietor: Alchemy Pharmatech Limited, Watlington, Oxfordshire OX49 5QJ (GB)
(72) Inventor: CLAYBOROUGH, Robert, Chelford Cheshire SK11 9SU (GB); HARRISON, Ian, Oxfordshire OX49 5EE (GB)
(74) Representative: HGF
(86) International application number: PCT/GB2022/052988
(87) International publication number: WO 2023/094821

(56) References cited:
- EP-B1- 2 271 388
- WO-A1-03/030973
- US-A- 4 137 914
- US-A1- 2008 092 887

## Description

This invention relates to a medicament delivery device, and in particular, to a medicament delivery device that includes a rupturable container that contains a medicament and a propellant.

### BACKGROUND

Medicament delivery devices facilitate the delivery of a dose of medicament to a user. In certain known medicament delivery devices, the medicament is stored in its liquid or solid phase and is suspended or otherwise mixed with a propellant in a container. Upon opening of the container, the propellant expands and/or vaporizes and is channeled towards the delivery site, such as the nasal cavity or mouth of the user. In doing so, the propellant carries the medicament such that the user receives a dose of the medicament.

For many medicaments, it is important that an accurate dose of medicament is delivered to the desired delivery site. In order to achieve this, a specific volume of medicament must be released from the container and the propellant must be released from the container in a manner that provides the necessary mechanism to deliver the complete dose of medicament to the delivery site. If the medicament comprises large solid particles, there is a risk that insufficient agitation of the medicament particles results in an inadequate dose of medicament being delivered to the delivery site. Another risk is that medicament particles clump together (e.g. in storage or transit of the medicament delivery device or container) and this detrimentally affects the accuracy of the dose delivered to the delivery site.

EP2271388B1 describes a particulate dispenser that is particularly suited for delivering a dose of medicament wherein a mechanism is provided for deaggregating and agitating a particulate so that it may be dispensed as a fluid.

It is an object of embodiments of the present invention to provide an improved medicament delivery device that overcomes one or more problems associated with prior art devices.

### BRIEF SUMMARY OF THE DISCLOSURE

In accordance with an aspect of the present invention there is provided a medicament delivery device comprising:
a casing defining a volume for receiving a rupturable container containing a medicament and a propellant; and
a needle hub assembly comprising a needle having a longitudinal axis, and a needle hub to which the needle is affixed, wherein the needle hub is engageable with the casing such that the needle extends into the volume for receiving the rupturable container;
wherein the needle hub has a profile such that circumferential interference between the needle hub and the casing prevents rotation of the needle hub relative to the casing about an axis parallel to the longitudinal axis when the needle hub is engaged with the casing.

In such embodiments, once the needle hub is engaged with the casing, relative rotation therebetween is prevented. Consequently, the orientation of the needle relative to the casing will not change during transit, storage or use of the device. This ensures that the needle remains in the desired orientation relative to the casing, as determined during assembly. Thus, provided that the needle is orientated for optimal rupturing of the rupturable container during assembly (e.g. so that a beveled end of the needle is in a particular orientation), this orientation will not be compromised during transit, storage or use of the medicament delivery device. As a result, the reliability of the medicament delivery device is improved, since the rupturable container may be ruptured at the same point across multiple uses of the same medicament delivery device and across multiple medicament delivery devices assembled in the same manner.

In certain embodiments, the needle hub may have a radially internal or external profile such that circumferential interference between the needle hub and the casing prevents rotation of the needle hub relative to the casing about an axis parallel to the longitudinal axis when the needle hub is engaged with the casing. This offers a particularly effective means for limiting relative rotation between the needle hub and the casing.

In certain embodiments, one of the needle hub and the casing may include a protrusion that extends radially relative to the longitudinal axis, and the other of the needle hub and the casing includes a recess for receiving the protrusion when the needle hub is engaged with the casing, wherein circumferential interference between the protrusion and the recess prevents rotation of the needle hub relative to the casing about an axis parallel to the longitudinal axis. In addition to preventing rotation of the needle hub relative to the casing, insertion of the protrusion in the recess additionally determines the rotational orientation of the needle hub relative to the casing in which the needle hub may be engaged with the casing. Such an embodiment ensures that assembly can only result in a medicament delivery device wherein the needle (e.g. and a beveled end of the needle) is in a desired/optimal orientation. In alternative embodiments, reference indicia (e.g. visual or tactile markers) may be used during assembly to ensure the desired orientation of the needle hub relative to the casing.

In certain embodiments, the protrusion may comprise an axially extending rib and the recess may comprise an axially extending slot for receiving the axially extending rib. This offers a particularly effective means for limiting relative rotation between the needle hub and the casing and determining a permitted orientation of the needle hub in which it may be engaged with the casing. The axially extending rib received in the recess may additionally stabilize the needle hub relative to the casing when engaged therewith.

In certain embodiments, the recess may include a stop that limits the axial extent to which the protrusion may be received in the recess. This may further improve the accuracy with which the needle hub may be assembled in the casing in the desired orientation.

In certain embodiments, the needle hub may be affixed to the casing, e.g. with an adhesive. These embodiments reduce any risk of undesirable movement of the needle hub relative to the casing following assembly.

In certain embodiments, the medicament delivery device may further comprise a cover that is engageable with the casing, wherein the cover may be moveable between a closed configuration in which the volume cannot be accessed, and an open configuration in which the volume can be accessed. The cover may be opened to permit insertion of a rupturable container in the volume, and closed following insertion of the rupturable container. The cover may subsequently be opened to remove an empty rupturable container once the contents thereof have been dispensed.

In certain embodiments, the cover may be manipulated by a user to cause a rupturable container received in the volume to move relative to the needle such that the needle may rupture the rupturable container. For example, the cover may be pressed so as to deform or otherwise move the rupturable container towards the needle so that it may be ruptured. Such embodiments offer a convenient means for actuating the medicament delivery device to deliver a dose of medicament to the user.

In certain embodiments, each of the needle and the needle hub comprise a bore such that medicament and propellant may pass therethrough and out of the medicament delivery device when the rupturable container is ruptured by the needle. The bore of the needle hub may have an internal diameter that increases in a direction out of the medicament delivery device. The increasing diameter of the bore facilitates the formation of a conic-shaped plume of medicament and propellant as it exits the medicament delivery device.

In certain embodiments, the casing comprises a plurality of guide ribs that guide at least a portion of the rupturable container towards the needle when a force is applied to the rupturable container. The guide ribs may improve the reliability of the medicament delivery device and/or improve the user experience when actuating the medicament delivery device.

In certain embodiments, the medicament delivery device may comprise a rupturable container received in the volume, wherein the rupturable container contains a medicament and a propellant. In certain embodiments, the propellant may be a gas or a liquid. In certain embodiments, the propellant may comprise one or more of: air, nitrogen, water, carbon dioxide, butane, hydrofluorocarbon (HFC), or hydrofluoroalkane (HFA).

In another aspect of the present invention, there is provided a method of assembling a medicament delivery device comprising the steps:
(i) providing a medicament delivery device according to any embodiment described above;
(ii) rotationally aligning the needle hub in a desired rotational alignment configuration relative to the casing; and
(iii) engaging the needle hub with the casing whilst the needle hub is in the desired rotational alignment configuration relative to the casing.

### BRIEF DESCRIPTION OF THE DRAWINGS

Embodiments of the invention are further described hereinafter with reference to the accompanying drawings, in which:
Figure 1 is a perspective, exploded, view of a medicament delivery device in accordance with an embodiment of the present invention;
Figure 2 is a cross-sectional side view of the medicament delivery device of Figure 1 in an assembled state;
Figure 3 is a perspective view of the needle hub of the medicament delivery device of Figures 1 and 2;
Figure 4 is a cross-sectional side view of the needle hub of Figure 3;
Figure 5 is a perspective view of the casing of the medicament delivery device of Figures 1 and 2;
Figure 6 shows a medicament delivery device with a needle hub according to an alternative embodiment of the present invention;
Figure 7 shows a cross section of the medicament delivery device of Figure 6, taken along the section line A-A of Figure 6;
Figure 8 shows a medicament delivery device with a needle hub according to a further alternative embodiment of the present invention;
Figure 9 shows a cross section of the medicament delivery device of Figure 8, taken along the section line B-B of Figure 8.

### DETAILED DESCRIPTION

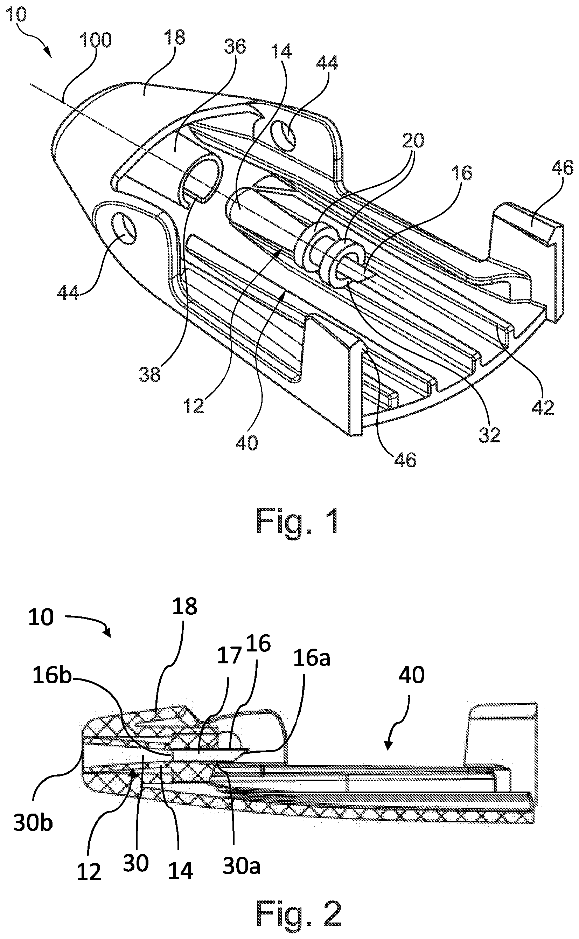

Figure 1 shows an exploded view of a medicament delivery device 10 in accordance with an embodiment of the present invention. The medicament delivery device 10 comprises a casing 18 defining a volume 40 for receiving a rupturable container (not shown) containing a medicament and a propellant, and further comprises a needle hub assembly 12. The needle hub assembly 12 comprises a needle 16 having a longitudinal axis 100, and a needle hub 14 to which the needle 16 is affixed. The needle hub 14 is engageable with the casing 18 such that the needle 16 extends into the volume 40 for receiving the rupturable container. In particular, the needle hub 14 is engageable in a boss 36 that is formed as part of the casing 18, as is described in further detail below.

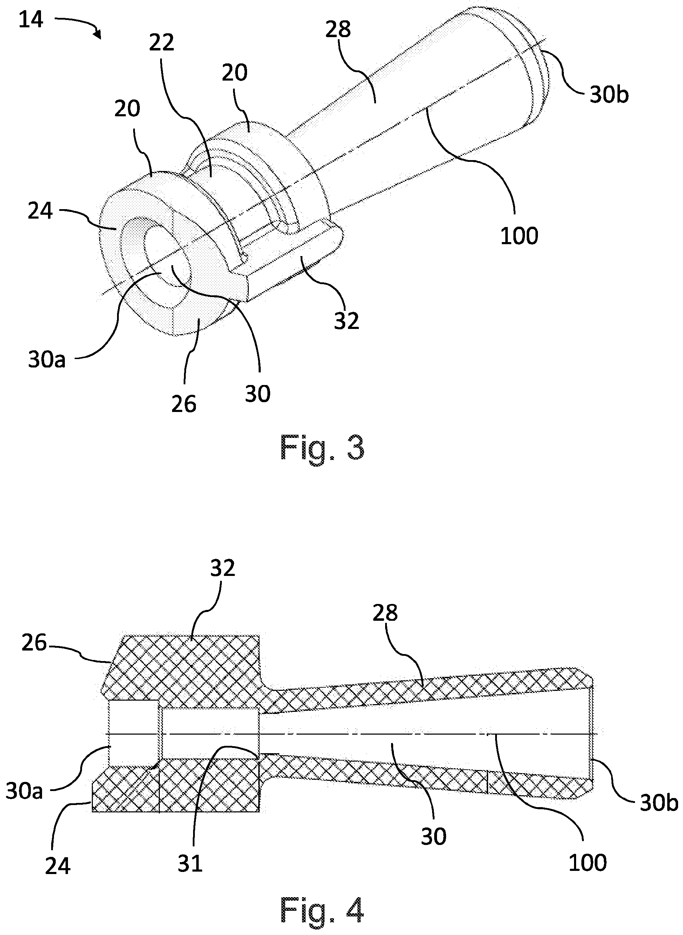

Figure 2 shows a cross-sectional side view of the medicament delivery device 10 of Figure 1. As shown in Figure 2, the needle hub 14 defines a through bore 30 having a first open end 30a that opens to the volume 40 and a second open end 30b that opens to the outside of the medicament delivery device 10. Surrounding the first open end 30a is a first end portion surface 24 and a second end portion surface 26 (see Figure 3). The plane of the first end portion surface 24 is substantially perpendicular to the longitudinal axis 100 while the plane of the second end portion surface 26 is inclined relative to the plane of the first end portion surface 24 and therefore tapers away from the first end portion surface 24.

The needle 16 is hollow by virtue of a needle bore 17 that extends along the longitudinal axis 100 through the needle 16 between a first end 16a and a second end 16b of the needle 16. The second end 16b of the needle 16 is attached to the first open end 30a of the through bore 30, as is described further below. Since the needle 16 is hollow, the assembled needle hub assembly 12 defines a continuous fluidic channel through the needle bore 17 and the through bore 30. The needle bore 17 and the through bore 30 pass through the boss 36 so as to provide a fluidic pathway from the volume 40 out of the medicament delivery device 10. The through bore 30 has a tapered profile such that its internal diameter increases along the longitudinal axis 100 in a direction towards the second open end 30b (i.e. along a direction out of the medicament delivery device 10).

The first end 16a of the needle 16 is beveled such that it includes a pointed tip. The beveled first end 16a is capable of rupturing a rupturable container disposed in the volume 40.

When assembled, the needle 16 extends into the through bore 30 through the first open end 30a. An internal shoulder 31 in the through bore 30 (see Figure 4) limits the axial distance by which the needle 16 may be inserted in the through bore 30.

A cover (not shown) may connect to the casing 18 and be moveable between a closed configuration and an open configuration so as to permit access to the volume (e.g. for inserting or removing the rupturable container). For example, the cover may be pivotally attached to a pair of pivot holes 44 and, in a closed configuration, may releasably engage with latches 46, where the latches 46 may be overcome by manual manipulation of the cover towards the open configuration or by pressing or squeezing the cover and/or the casing 18 to release the latches 46.

In use, the rupturable container is moved towards the needle 16 so that the beveled first end 16a of the needle 16 contacts and ruptures the rupturable container. As a result, the propellant within the rupturable container vaporises and/or rapidly expands thereby creating a flow of propellant and medicament entrained therein through the needle bore 17 and the through bore 30 out of the medicament delivery device 10. Due to the tapered internal profile of the through bore 30, the released propellant and medicament form a conic-shaped plume as they exit the medicament delivery device 10. The plume may facilitate the spreading of the dose of medicament over a large surface area (e.g. the lining of the nasal cavity) so as to maximise absorption of the medicament by the user. The medicament delivery device 10 may be shaped or otherwise configured so that the exiting medicament may travel into any suitable cavity of the user's body, including but not limited to the nose and the mouth.

The rupturable container may be moved relative to the needle 16 by any suitable means. In certain preferable embodiments, the rupturable container is made of a pliable material and, upon actuation of the medicament delivery device, is caused to deform so as to contact the needle 16. For example, a part of the cover (or another component of the medicament delivery device 10) may be manipulated (e.g. deformed) so that it urges against the rupturable container and at least part of the rupturable container is consequently urged towards the needle 16.

In the non-limiting embodiment shown in the Figures, the casing 18 is provided with a plurality of guide ribs 42 in the volume 40. The guide ribs 42 each have a height that reduces along a direction towards the assembled needle 16. In use, the rupturable container is received in the volume 40 so that it rests on the guide ribs 42. When a force is applied to the rupturable container by a user (e.g. it is compressed by deformation of a cover as described above), at least a portion of the rupturable container is guided by the profile of the guide ribs 42 towards the needle 16 so as to ensure that the needle 16 ruptures the rupturable container.

The needle hub 14 is described in further detail below with reference to Figures 3 and 4. Figure 3 shows a perspective view of the needle hub 14 and Figure 4 shows a cross-sectional side view of the needle hub 14. As shown in Figure 3, the needle hub 14 has a flared portion 28 and a mounting region 20 that is configured to be received within and generally conform to the internal profile of the boss 36. In the non-limiting embodiment shown in the Figures, the boss 36 has a generally cylindrical internal profile and so the mounting region 20 has a complementary circular cross section. In the embodiment shown in Figure 3, the mounting region 20 comprises a pair of axially spaced flanges 20 defined by an intermediate annulus 22 therebetween. The flanges 20 and the annulus 22 are not circumferentially continuous as they are interrupted by an axially extending rib 32 that extends radially outward from the longitudinal axis 100 to a greater extent relative to the flanges 20. The mounting region (flanges 20 in the embodiment shown in the Figures) provides a surface that may be bonded or otherwise fixed to the casing 18 (i.e. the inside of the boss 36 in the embodiment shown in the Figures), e.g. by an adhesive or other bonding agent during assembly.

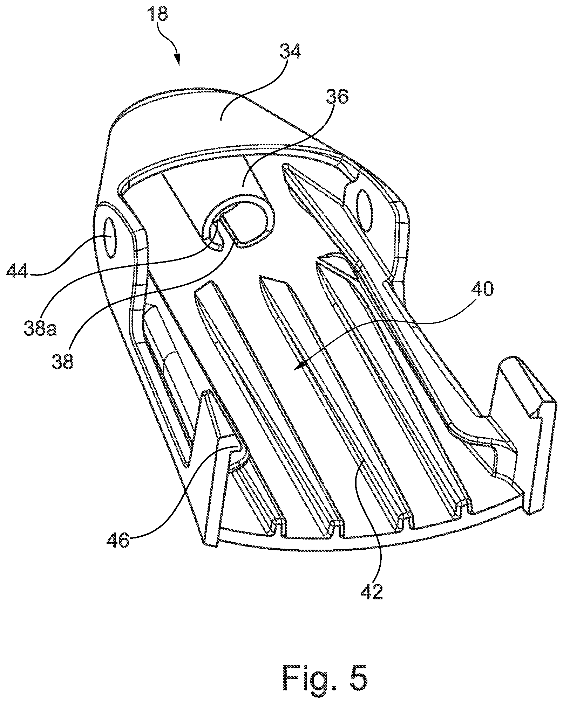

The axially extending rib 32 provides a radially outwardly extending protrusion that defines a non-circular radially outer surface in cross section of the needle hub 14. During assembly, the axially extending rib 32 is received in a recess in the form of an axial slot 38 (shown in Figures 1 and 5) in the boss 36 so that, when received, rotation of the needle hub 14 relative to the casing 18 is prevented. Moreover, the needle hub 14 may only be inserted into the boss 36 when the axially extending rib 32 is aligned with the axial slot 38 thereby permitting only a single rotational orientation of the needle hub 14 when assembled with the casing 18. Thus, in addition to the prevention of relative rotation, the engagement of the axially extending rib 32 in the axial slot 38 ensures that the needle hub assembly 12 can only be assembled in the casing in a single, particular orientation. As such, the rotational alignment of the needle 16, and its beveled first end 16a will be consistent relative to the casing 18 from one assembled device 10 to the next and will not change during transit, storage or handling of the medicament delivery device 10. This may advantageously ensure that the beveled first end 16a of the needle 16 contacts a rupturable container received in the volume 40 at a specific predetermined point that is consistent from one assembled device 10 to the next. Consequently, the consistency of the rupturing of the rupturable container may be improved, thereby improving the likelihood that the desired dose of medicament is delivered to the user.

In alternative embodiments, the needle hub 14 may have any alternative profile, external or otherwise, such that circumferential interference between the needle hub 14 and the casing 18 prevents rotation of the needle hub 14 relative to the casing 18 about an axis parallel to the longitudinal axis 100 when the needle hub 14 is engaged with the casing 18. For example, the needle hub 14 may have any non-circular external profile (in cross-section) and may be received in a complementary recess defined in the casing 18. In some embodiments, the needle hub 14 may form a female connector part that has a non-circular internal profile, and that may receive a complementary male connector part (albeit that is hollow) of the casing 18.

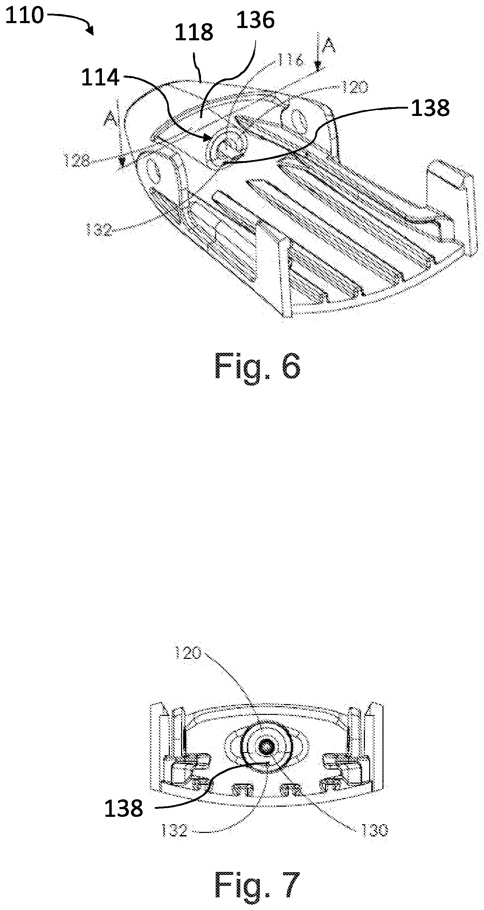

Examples of such alternative embodiments are described below with reference to Figures 6 to 9. Figure 6 shows an alternative embodiment of a medicament delivery device 110 with a needle hub 114 that shares many of the features of the medicament delivery device 10 and needle hub 14 described above. Reference numerals that are transposed by 100 are used to identify common or similar features of the medicament delivery device 110 and the needle hub 114 relative to the medicament delivery device 10 and the needle hub 14. In particular, amongst other common features, the needle hub 114 defines a bore 130 and comprises a mounting region 120, and has a needle 116 attached thereto. The mounting portion 120 is configured to be received in and generally conform to the internal profile of a boss 136 formed as part of a casing 118 of the medicament delivery device 110. The boss 136 has a generally cylindrical internal profile and is capable of receiving the mounting region 120 that has a complementary circular cross section. The mounting portion 120 may be bonded or otherwise fixed to the boss 136 to secure the needle hub 114 to the casing 118. An axially extending rib 132 extends radially inwardly from the boss 136. A complementary axial slot 138 is provided in the mounting region 120 and defines a non-circular radially outer surface in cross section (shown in Figure 7, which corresponds to section A-A of Figure 6) of the needle hub 114. During assembly, the axially extending rib 132 is received in the axial slot 138 so that, when received, rotation of the needle hub 114 relative to the casing 118 is prevented. Moreover, the needle hub 114 may only be inserted into the boss 136 when the axially extending rib 132 is aligned with the axial slot 138 thereby permitting only a single rotational orientation of the needle hub 114 when assembled with the casing 118. Thus, in addition to the prevention of relative rotation, the engagement of the axially extending rib 132 in the axial slot 138 ensures that the needle hub assembly can only be assembled in the casing 118 in a single, particular orientation.

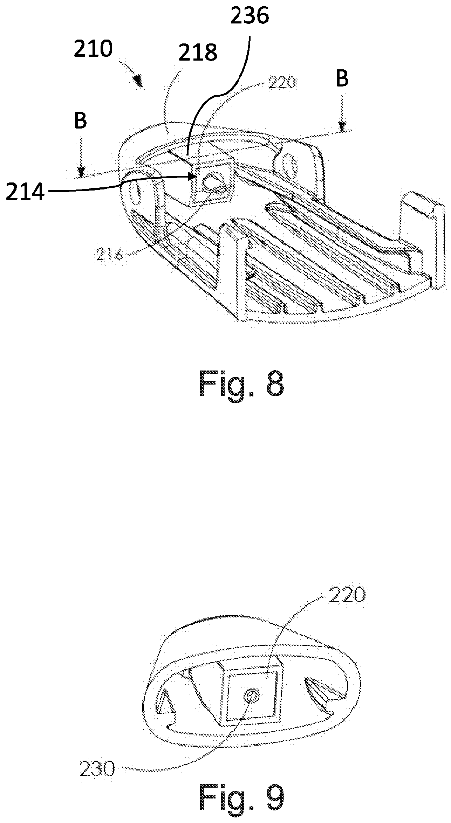

Figure 8 shows an alternative embodiment of a medicament delivery device 210 having a needle hub 214 that shares many of the features of the medicament delivery device 10 and needle hub 14 described above. Reference numerals that are transposed by 200 are used to identify common or similar features of the medicament delivery device 210 and needle hub 214 relative to the medicament delivery device 10 and needle hub 14. In particular, amongst other common features, the needle hub 214 defines a bore 230 and comprises a mounting region 220, and has a needle 216 attached thereto. The mounting portion 220 is configured to be received within and generally conform to the internal profile of a boss 236 formed as part of a casing 218 of the medicament delivery device 210. The boss 236 has a generally square internal profile and is capable of receiving the mounting region 220 that has a complementary square cross section. The mounting portion 220 may be bonded or otherwise fixed to the boss 236 to secure the needle hub 214 to the casing 218. The square profile of the mounting portion 220 defines a non-circular radially outer surface in cross section (shown in Figure 9, which corresponds to section B-B of Figure 8) of the needle hub 214. Engagement between the square profile of the mounting portion 220 and the complementary internal profile of the boss 236 prevents relative rotation of the needle hub 214 and the boss 236 when the needle hub 214 is mounted thereto. Moreover, the needle hub 214 may only be inserted into the boss 236 when the square profile of the mounting portion 220 is aligned with the square inner profile of the boss 236. For a square, therefore, four possible alignment orientations may be possible.

In alternative embodiments, the mounting portion may have any non-circular radially outer or inner surface cross section that is receivable inside/over a boss having a complementary inner/outer profile. In certain embodiments, the non-circular radially outer or inner surface cross section may be polygonal, including but not limited to square or rectangular. In certain embodiments, one of the mounting portion or the boss may comprise an axial rib or other protrusion whilst the other may comprise a complementary slot or recess. In certain embodiments, the needle hub may only be engageable with the boss in a single relative rotational orientation. As noted above, in other embodiments, the needle hub may be engageable with the boss in more than one relative rotational orientation. In certain embodiments, reference indicia may be provided for assisting the assembler with assembly, so that the needle hub (and hence needle) are orientated in the desired orientation relative to the boss and casing.

As described above, in certain embodiments, means for ensuring or guiding correct rotational alignment of the needle hub 14 relative to the casing 18 may be provided. In the above-described non-limiting embodiment, such means are provided by the axially extending rib 32 (forming a radially extending protrusion) and the axial slot 38 since only one relative rotational orientation is permitted when the needle hub 14 is received by the casing 18.

In alternative embodiments, any similar key and keyhole-like arrangement may be employed to achieve correct or desired rotational alignment. In general, one of the needle hub 14 and the casing 18 may include a protrusion that extends radially relative to the longitudinal axis 100, and the other of the needle hub 14 and the casing 18 includes a recess for receiving the protrusion when the needle hub 14 is engaged with the casing 18, wherein circumferential interference between the protrusion and the recess prevents rotation of the needle hub 14 relative to the casing 18 about an axis parallel to the longitudinal axis 100. In certain embodiments, the recess may comprise a stop that limits the extent to which the protrusion may be inserted into the recess. Thus, the position of the needle hub 14 relative to the casing 18 may be inhibited along a further direction, thereby further ensuring that the needle hub 14 (and hence needle 16) is in the desired configuration relative to the casing 18 (and the rupturable container that may be received therein).

In certain embodiments, multiple rotational alignments of the needle hub 14 relative to the casing 18 may be possible (e.g. if the needle hub 14 has a square cross-section and is received in a complementary recess in the casing 18), and reference indicia (e.g. visual or tactile alignment markers) may be provided to facilitate correct or desired rotational alignment during assembly of the needle hub 14 in the casing 18.

In certain embodiments the propellant may be any sufficiently mobile fluid that may agitate the medicament (e.g. if in a particulate state) into turbulent flow. The propellant is further preferably stable during storage and inert to the medicament and the delivery site. In certain embodiments, the propellant may be a gas or a liquid. Non-limiting examples of propellants that may be used in certain embodiments of the present invention include air, nitrogen, water, or conventional optionally fluorinated lower hydrocarbon propellants such as hydrofluorocarbon (HFC), carbon dioxide, butane, hydrofluoroalkane (HFA) propellants or any compatible combination thereof.

Throughout the description and claims of this specification the term radial internal profile or radially inner surface refers to a surface that faces radially inwardly. Similarly, the term radial external profile or radially outer surface refers to a surface that faces radially outwardly.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

The scope of the present invention is defined by the claims that follow.

## Claims

1. A medicament delivery device (10) comprising:
a casing (18) defining a volume (40) for receiving a rupturable container containing a medicament and a propellant; and
a needle hub assembly (12) comprising a needle (16) having a longitudinal axis (100), and a needle hub (14) to which the needle (16) is affixed, wherein the needle hub (14) is engageable with the casing (18) such that the needle (16) extends into the volume (40) for receiving the rupturable container;
**characterised in that** the needle hub (14) has a profile such that circumferential interference between the needle hub (14) and the casing (18) prevents rotation of the needle hub (14) relative to the casing (18) about an axis parallel to the longitudinal axis (100) when the needle hub (14) is engaged with the casing (18).

2. A medicament delivery device (10) according to claim 1, where the needle hub (14) has a radial internal or external profile such that circumferential interference between the needle hub (14) and the casing (18) prevents rotation of the needle hub (14) relative to the casing (18) about an axis parallel to the longitudinal axis (100) when the needle hub (14) is engaged with the casing (18).

3. A medicament delivery device (10) according to claim 1 or 2, wherein one of the needle hub (14) and the casing (18) includes a protrusion that extends radially relative to the longitudinal axis (100), and the other of the needle hub (14) and the casing (18) includes a recess for receiving the protrusion when the needle hub (14) is engaged with the casing (18), wherein circumferential interference between the protrusion and the recess prevents rotation of the needle hub (14) relative to the casing (18) about an axis parallel to the longitudinal axis (100).

4. A medicament delivery device (10) according to claim 3, wherein the protrusion comprises an axially extending rib (32) and the recess comprises an axially extending slot (38) for receiving the axially extending rib (32).

5. A medicament delivery device (10) according to claim 3 or 4, wherein the recess includes a stop that limits the axial extent to which the protrusion may be received in the recess.

6. A medicament delivery device (10) according to any preceding claim, wherein the needle hub (14) is affixed to the casing (18).

7. A medicament delivery device (10) according to claim 6, wherein the needle hub (14) is affixed to the casing (18) with an adhesive.

8. A medicament delivery device (10) according to any preceding claim, further comprising a cover that is engageable with the casing (18), and wherein the cover is moveable between a closed configuration in which the volume (40) cannot be accessed, and an open configuration in which the volume (40) can be accessed.

9. A medicament delivery device (10) according to claim 8, wherein the cover may be manipulated by a user to cause a rupturable container received in the volume (40) to move relative to the needle (16) such that the needle (16) may rupture the rupturable container.

10. A medicament delivery device (10) according to any preceding claim, wherein each of the needle (16) and the needle hub (14) comprise a bore (17, 30) such that medicament and propellant may pass therethrough and out of the medicament delivery device (10) when the rupturable container is ruptured by the needle (16);
optionally wherein the bore (30) of the needle hub (14) has an internal diameter that increases in a direction out of the medicament delivery device (10).

11. A medicament delivery device (10) according to any preceding claim, wherein the casing (18) comprises a plurality of guide ribs (42) that guide at least a portion of the rupturable container to towards the needle (16) when a force is applied to the rupturable container.

12. A medicament delivery device (10) according to any preceding claim, comprising a rupturable container received in the volume (40), wherein the rupturable container contains a medicament and a propellant.

13. A medicament delivery device (10) according to claim 12, wherein the propellant is a gas or a liquid.

14. A medicament delivery device (10) according to claim 12 or 13, wherein the propellant comprises one or more of: air, nitrogen, water, carbon dioxide, butane, hydrofluorocarbon (HFC), or hydrofluoroalkane (HFA).

15. A method of assembling a medicament delivery device (10) comprising the steps:
(i) providing a medicament delivery device (10) according to any preceding claim;
(ii) rotationally aligning the needle hub (14) in a desired rotational alignment configuration relative to the casing (18); and
(iii) engaging the needle hub (14) with the casing (18) whilst the needle hub (14) is in the desired rotational alignment configuration relative to the casing (18).

## Patentansprüche

1. Medikamentenabgabevorrichtung (10), umfassend:
ein Gehäuse (18), das ein Volumen (40) zum Aufnehmen eines aufbrechbaren Behälters definiert, der ein Medikament und ein Treibmittel enthält; und
eine Nadelnabenbaugruppe (12), die eine Nadel (16) mit einer Längsachse (100) und eine Nadelnabe (14), an der die Nadel (16) befestigt ist, umfasst, wobei die Nadelnabe (14) mit dem Gehäuse (18) in Eingriff bringbar ist, sodass sich die Nadel (16) in das Volumen (40) zum Aufnehmen des aufbrechbaren Behälters erstreckt;
**dadurch gekennzeichnet, dass** die Nadelnabe (14) ein Profil aufweist, sodass umlaufende Interferenz zwischen der Nadelnabe (14) und dem Gehäuse (18) Drehung der Nadelnabe (14) relativ zu dem Gehäuse (18) um eine Achse parallel zu der Längsachse (100) verhindert, wenn die Nadelnabe (14) mit dem Gehäuse (18) in Eingriff ist.

2. Medikamentenabgabevorrichtung (10) nach Anspruch 1, wobei die Nadelnabe (14) ein radiales Innen- oder Außenprofil aufweist, sodass umlaufende Interferenz zwischen der Nadelnabe (14) und dem Gehäuse (18) Drehung der Nadelnabe (14) relativ zu dem Gehäuse (18) um eine Achse parallel zu der Längsachse (100) verhindert, wenn die Nadelnabe (14) mit dem Gehäuse (18) in Eingriff ist.

3. Medikamentenabgabevorrichtung (10) nach Anspruch 1 oder 2, wobei eines von der Nadelnabe (14) und dem Gehäuse (18) einen Vorsprung beinhaltet, der sich radial relativ zu der Längsachse (100) erstreckt, und das andere von der Nadelnabe (14) und dem Gehäuse (18) eine Aussparung zum Aufnehmen des Vorsprungs beinhaltet, wenn die Nadelnabe (14) mit dem Gehäuse (18) in Eingriff ist, wobei umlaufende Interferenz zwischen dem Vorsprung und der Aussparung Drehung der Nadelnabe (14) relativ zu dem Gehäuse (18) um eine Achse parallel zu der Längsachse (100) verhindert.

4. Medikamentenabgabevorrichtung (10) nach Anspruch 3, wobei der Vorsprung eine sich axial erstreckende Rippe (32) umfasst und die Aussparung einen sich axial erstreckenden Schlitz (38) zum Aufnehmen der sich axial erstreckenden Rippe (32) umfasst.

5. Medikamentenabgabevorrichtung (10) nach Anspruch 3 oder 4, wobei die Aussparung einen Anschlag beinhaltet, der das axiale Ausmaß begrenzt, in dem der Vorsprung in der Aussparung aufgenommen sein kann.

6. Medikamentenabgabevorrichtung (10) nach einem vorhergehenden Anspruch, wobei die Nadelnabe (14) an dem Gehäuse (18) befestigt ist.

7. Medikamentenabgabevorrichtung (10) nach Anspruch 6, wobei die Nadelnabe (14) an dem Gehäuse (18) mit einem Klebstoff befestigt ist.

8. Medikamentenabgabevorrichtung (10) nach einem vorhergehenden Anspruch, ferner umfassend eine Abdeckung, die mit dem Gehäuse (18) in Eingriff bringbar ist, und wobei die Abdeckung zwischen einer geschlossenen Konfiguration, in der auf das Volumen (40) nicht zugegriffen werden kann, und einer offenen Konfiguration, in der auf das Volumen (40) zugegriffen werden kann, bewegbar ist.

9. Medikamentenabgabevorrichtung (10) nach Anspruch 8, wobei die Abdeckung durch einen Benutzer manipuliert werden kann, um zu bewirken, dass sich ein aufbrechbarer Behälter, der in dem Volumen (40) aufgenommen ist, relativ zu der Nadel (16) bewegt, sodass die Nadel (16) den aufbrechbaren Behälter aufbrechen kann.

10. Medikamentenabgabevorrichtung (10) nach einem vorhergehenden Anspruch, wobei jedes von der Nadel (16) und der Nadelnabe (14) eine Bohrung (17, 30) umfasst, sodass Medikament und Treibmittel dort hindurch und aus der Medikamentenabgabevorrichtung (10) gelangen können, wenn der aufbrechbare Behälter durch die Nadel (16) aufgebrochen wird;
wobei optional die Bohrung (30) der Nadelnabe (14) einen Innendurchmesser aufweist, der in einer Richtung aus der Medikamentenabgabevorrichtung (10) zunimmt.

11. Medikamentenabgabevorrichtung (10) nach einem vorhergehenden Anspruch, wobei das Gehäuse (18) eine Vielzahl von Führungsrippen (42) umfasst, die zumindest einen Abschnitt des aufbrechbaren Behälters zu der Nadel (16) führt, wenn eine Kraft auf den aufbrechbaren Behälter ausgeübt wird.

12. Medikamentenabgabevorrichtung (10) nach einem vorhergehenden Anspruch, umfassend einen aufbrechbaren Behälter, der in dem Volumen (40) aufgenommen ist, wobei der aufbrechbare Behälter ein Medikament und ein Treibmittel enthält.

13. Medikamentenabgabevorrichtung (10) nach Anspruch 12, wobei das Treibmittel ein Gas oder eine Flüssigkeit ist.

14. Medikamentenabgabevorrichtung (10) nach Anspruch 12 oder 13, wobei das Treibmittel eines oder mehrere von Folgendem umfasst: Luft, Stickstoff, Wasser, Kohlendioxid, Butan, Fluorkohlenwasserstoff (HFC) oder Hydrofluoralkan (HFA).

15. Verfahren zum Zusammenbauen einer Medikamentenabgabevorrichtung (10), umfassend die folgenden Schritte:
(i) Bereitstellen einer Medikamentenabgabevorrichtung (10) nach einem vorhergehenden Anspruch;
(ii) Drehausrichten der Nadelnabe (14) in einer gewünschten Drehausrichtungskonfiguration relativ zu dem Gehäuse (18); und
(iii) Ineingriffbringen der Nadelnabe (14) mit dem Gehäuse (18), während die Nadelnabe (14) in der gewünschten Drehausrichtungskonfiguration relativ zu dem Gehäuse (18) ist.

## Revendications

1. Dispositif d'administration de médicament (10) comprenant :
un boîtier (18) définissant un volume (40) destiné à recevoir un récipient pouvant être rompu contenant un médicament et un propulseur ; et
un assemblage d'embase d'aiguille (12) comprenant une aiguille (16) présentant un axe longitudinal (100), et une embase d'aiguille (14) à laquelle l'aiguille (16) est fixée, ladite embase d'aiguille (14) pouvant venir en prise avec le boîtier (18) de sorte que l'aiguille (16) s'étende dans le volume (40) destiné à recevoir le récipient pouvant être rompu ;
**caractérisé en ce que** l'embase d'aiguille (14) présente un profil tel qu'une interférence circonférentielle entre l'embase d'aiguille (14) et le boîtier (18) empêche la rotation de l'embase d'aiguille (14) par rapport au boîtier (18) autour d'un axe parallèle à l'axe longitudinal (100) lorsque l'embase d'aiguille (14) vient en prise avec le boîtier (18).

2. Dispositif d'administration de médicament (10) selon la revendication 1, ladite embase d'aiguille (14) présentant un profil interne ou externe radial de sorte qu'une interférence circonférentielle entre l'embase d'aiguille (14) et le boîtier (18) empêche la rotation de l'embase d'aiguille (14) par rapport au boîtier (18) autour d'un axe parallèle à l'axe longitudinal (100) lorsque l'embase d'aiguille (14) est en prise avec le boîtier (18).

3. Dispositif d'administration de médicament (10) selon la revendication 1 ou 2, un élément parmi l'embase d'aiguille (14) et le boîtier (18) comprenant une saillie qui s'étend radialement par rapport à l'axe longitudinal (100), et l'autre élément parmi l'embase d'aiguille (14) et le boîtier (18) comprenant un évidement destiné à recevoir la saillie lorsque l'embase d'aiguille (14) vient en prise avec le boîtier (18), une interférence circonférentielle entre la saillie et l'évidement empêchant la rotation de l'embase d'aiguille (14) par rapport au boîtier (18) autour d'un axe parallèle à l'axe longitudinal (100).

4. Dispositif d'administration de médicament (10) selon la revendication 3, ladite saillie comprenant une nervure s'étendant axialement (32) et ledit évidement comprenant une fente s'étendant axialement (38) destinée à recevoir ladite nervure s'étendant axialement (32).

5. Dispositif d'administration de médicament (10) selon la revendication 3 ou 4, ledit évidement comprenant une butée qui limite l'étendue axiale à laquelle la saillie peut être reçue dans l'évidement.

6. Dispositif d'administration de médicament (10) selon l'une quelconque des revendications précédentes, ladite embase d'aiguille (14) étant fixée au boîtier (18).

7. Dispositif d'administration de médicament (10) selon la revendication 6, ladite embase d'aiguille (14) étant fixée au boîtier (18) avec un adhésif.

8. Dispositif d'administration de médicament (10) selon l'une quelconque des revendications précédentes, comprenant en outre un couvercle qui peut être mis en prise avec le boîtier (18), et ledit couvercle pouvant être déplacé entre une configuration fermée dans laquelle le volume (40) n'est pas accessible, et une configuration ouverte dans laquelle le volume (40) est accessible.

9. Dispositif d'administration de médicament (10) selon la revendication 8, ledit couvercle pouvant être manipulé par un utilisateur de manière à provoquer le déplacement d'un récipient pouvant être rompu reçu dans le volume (40) par rapport à l'aiguille (16) de sorte que l'aiguille (16) puisse rompre le récipient pouvant être rompu.

10. Dispositif d'administration de médicament (10) selon l'une quelconque des revendications précédentes, ladite aiguille (16) et ladite embase d'aiguille (14) comprenant chacune un alésage (17, 30) de sorte que le médicament et le propulseur puissent passer à travers le dispositif d'administration de médicament (10) et hors de celui-ci lorsque le récipient pouvant être rompu est rompu par l'aiguille (16) ;
éventuellement, ledit alésage (30) de l'embase d'aiguille (14) présentant un diamètre interne qui augmente suivant une direction hors du dispositif d'administration de médicament (10).

11. Dispositif d'administration de médicament (10) selon l'une quelconque des revendications précédentes, ledit boîtier (18) comprenant une pluralité de nervures de guidage (42) qui guident au moins une partie du récipient pouvant être rompu en direction de l'aiguille (16) lorsqu'une force est appliquée sur le récipient pouvant être rompu.

12. Dispositif d'administration de médicament (10) selon l'une quelconque des revendications précédentes, comprenant un récipient pouvant être rompu reçu dans le volume (40), ledit récipient pouvant être rompu contenant un médicament et un propulseur.

13. Dispositif d'administration de médicament (10) selon la revendication 12, ledit propulseur étant un gaz ou un liquide.

14. Dispositif d'administration de médicament (10) selon la revendication 12 ou 13, ledit propulseur comprenant un ou plusieurs éléments parmi : l'air, l'azote, l'eau, le dioxyde de carbone, le butane, un hydrofluorocarbone (HFC) ou un hydrofluoroalcane (HFA).

15. Procédé d'assemblage d'un dispositif d'administration de médicament (10) comprenant les étapes :
(i) la fourniture d'un dispositif d'administration de médicament (10) selon l'une quelconque des revendications précédentes ;
(ii) l'alignement rotatif de l'embase d'aiguille (14) dans une configuration d'alignement rotatif souhaitée par rapport au boîtier (18) ; et
(iii) la mise en prise de l'embase d'aiguille (14) avec le boîtier (18) lorsque l'embase d'aiguille (14) se trouve dans la configuration d'alignement rotatif souhaitée par rapport au boîtier (18).
